# EUROPEAN PATENT SPECIFICATION

(11) **EP 0 834 333 B1**
(45) Date of publication and mention of the grant of the patent: **02.07.2003**
(21) Application number: 97307782.9
(22) Date of filing: 02.10.1997
(51) Int. Cl.: A61M 25/10, A61N 5/10

(54) **Centering balloon catheter**
Ballonkatheter zur Zentrierung
Cathéter de ballon pour le centrage

(30) Priority: 03.10.1996 US 725364
(43) Date of publication of application: 08.04.1998
(73) Proprietor: Cordis Corporation, Miami Lakes Florida 33014 (US)
(72) Inventor: Dunham, Susan L., Pembroke Pines, Florida 33026 (US); Leone, James E., Miami, Florida 33156 (US)
(74) Representative: Mercer, Christopher Paul

(56) References cited:
- EP-A- 0 261 831
- EP-A- 0 688 580
- WO-A-96/10436
- WO-A-96/14898
- US-A- 5 447 497

## Description

The present invention relates to medical catheters, such as balloon catheters. The present invention has even further relation to such catheters which are designed to center a lumen of the catheter within a body vessel.

Restenosis after arterial intervention in general, and after percutaneous transluminal coronary angioplasty ("PTCA") in particular, is a primary concern of physicians practicing PTCA today. Conventional PTCA is performed using a standard balloon catheter such as the type described in U.S. Patent 5,304,197 issued to Pinchuk et al. on April 19, 1994. Balloon catheters are typically used with a guidewire which is inserted into the patient's artery until its distal end is advanced past the diseased or stenotic area of the vessel, where there is a buildup of material. Balloon catheters typically have a guidewire lumen so that the proximal end of the guidewire can be inserted into the distal end of the balloon catheter. Thereafter, the balloon catheter is advanced over the guidewire until the balloon is adjacent the buildup of material. The balloon is then inflated to compress the buildup. Finally, the balloon is deflated and the catheter is pulled back up the guidewire and removed from the patient's artery. Restenosis of the artery often occurs after this procedure. That is the same area of the vessel collapses or becomes clogged again.

Recent technology has discovered that treating the diseased area of the vessel with radiation, after balloon angioplasty, helps prevent restenosis. Such technology is described in U.S. Patent 5,199,939 issued to Dake et al. on April 6, 1993. Current technology contemplates the delivery of unspecified doses of radiation via wires having radioactive distal tips. A catheter would be inserted into the vasculature and advanced to the site of the previous angioplasty. The radioactive source wire would then be advanced through a lumen in the catheter so that its radioactive tip is adjacent the diseased site and can deliver the requisite amount of radiation. Thereafter the catheter and wire are removed. Such a device is described in PCT Application PCT/US94/04857 having an international publication number WO 94/25106 and publication date of November 10, 1994.

Because the intensity of the radiation delivered to the vessel wall varies in inverse proportion to the square of the distance between the radioactive source and the vessel wall, it is desirable to center the radioactive wire within the vessel. This is also true when exposing a vessel to a light source. This prevents areas of the vessel from being overexposed or underexposed to the radiation. One such way to center the radioactive wire would be to deliver the wire to the site via a central lumen of a spiral balloon catheter. An example of a spiral catheter is given in U.S. Patent 4,762,130 issued to Fogarty et al. on August 9,1988.

When using a spiral balloon in a vessel, the balloon is wrapped in a spiral fashion around a centering lumen. Likewise, a balloon could be molded in a spiral shape around the centering lumen. When inflated, the outer surface, or major diameter of the balloon pushes against the vessel wall, while the inner surface, or minor diameter, of the balloon pushes the centering lumen towards the center of the vessel. This centering effect increases as the pitch of the turns of the spiral balloon decreases.

Another example of a centering catheter is disclosed in European Patent Application number 94109858.4 filed on June 24, 1994 by Schneider (Europe) AG and published as EP-A-0 688 580. This type of catheter is often referred to as a segmented balloon catheter. It centers a centering lumen using the same principle as the spiral balloon catheter. However, instead of having a spiral it has a series of peaks and valleys created by segmenting an ordinary balloon catheter. Features of the present invention known from this prior art catheter have been placed in the preamble of claim 1 appended hereto.

When delivering a radiation or light source to a vessel, an angioplasty operation is typically performed first. That is a physician first inserts an angioplasty balloon to the diseased portion of the artery and inflates the balloon to compress any deposits that have built up there. Thereafter, the physician removes the angioplasty balloon catheter and inserts the centering catheter. Centering catheters, such as spiral and segmented catheters, are impractical for performing angioplasty because they have areas which do not contact the vessel wall, thereby leaving un-compressed deposits within the vessel. There has been a desire to have a single catheter which can perform an angioplasty and, thereafter, can deliver and center a radioactive source wire. This would relieve the physician from having to remove and replace catheters during the procedure. The present invention is intended to provide such a catheter.

### SUMMARY OF THE INVENTION

In accordance with the present invention, there is provided a catheter for insertion into a vessel of a patient. The catheter has distal and proximal ends and includes an elongated flexible outer shaft and an elongated flexible inner shaft. The inner shaft has a central lumen extending therethrough. The catheter has first and second inflation lumens extending through at least one of the inner and outer shafts. The catheter includes an inflatable outer balloon disposed at its distal end. The outer balloon is in fluid communication with the first inflation lumen for inflation and deflation thereof. The catheter further includes an inflatable inner balloon disposed within the outer balloon. The inner balloon is in fluid communication with the second inflation lumen for inflation and deflation thereof. The inner shaft of the catheter extends through the inner balloon. The inner balloon is a centering balloon and is able to center the distal end of the inner shaft within the vessel once it is inflated.

### BRIEF DESCRIPTION OF THE DRAWINGS

While the specification concludes with claims which particularly point out and distinctly claim the subject matter forming the present invention, it is believed that the invention will be better understood from the following description of the preferred embodiment taken in conjunction with the accompanying drawings wherein:
Figure 1 is a simplified cross-sectional view of a distal end of a catheter made in accordance with the present invention wherein the outer balloon is inflated and the inner balloon is deflated.
Figure 2 is a cross-sectional view of the distal end of a balloon catheter made in accordance with the present invention, similar to that of Figure 1, but showing a cross-section of the inner balloon as well, and also showing the inner balloon inflated.
Figure 3 is a cross-sectional view of the catheter shown in Figure 1, taken along line 3-3.
Figure 4 is a cross-sectional view of the catheter shown in Figure 1, taken along line 4-4.
Figure 5 is a cross-sectional view of the catheter shown in Figure 1, taken along line 5-5.
Figure 6 is a view similar to that of Figure 2 but showing an alternative embodiment of the present invention.
Figure 7 is a view similar to that of Figure 2 but showing yet another alternative embodiment of the present invention.

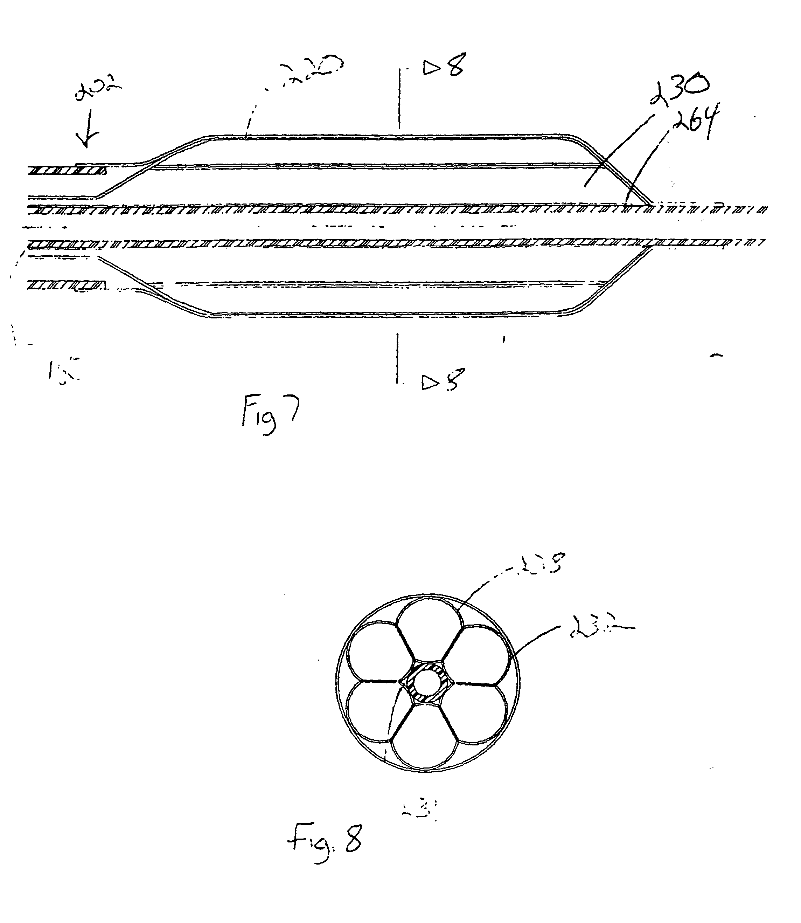

Figure 8 is a cross-sectional view of the catheter shown in Figure 7 taken along lines 8-8 and showing the centering catheter in its inflated state.
Figure 9 is a view similar to that of Figure 1 but showing an alternative embodiment of the present invention.
Figure 10 is a cross-sectional view of the catheter shown in Figure 9 taken along line 10-10.
Figure 11 is a cross-sectional view of the catheter shown in Figure 9 taken along line 11-11.

### DETAILED DESCRIPTION OF THE INVENTION

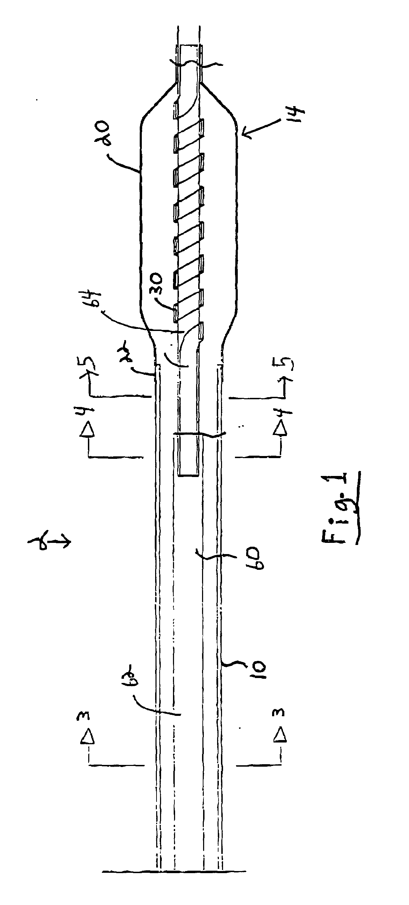

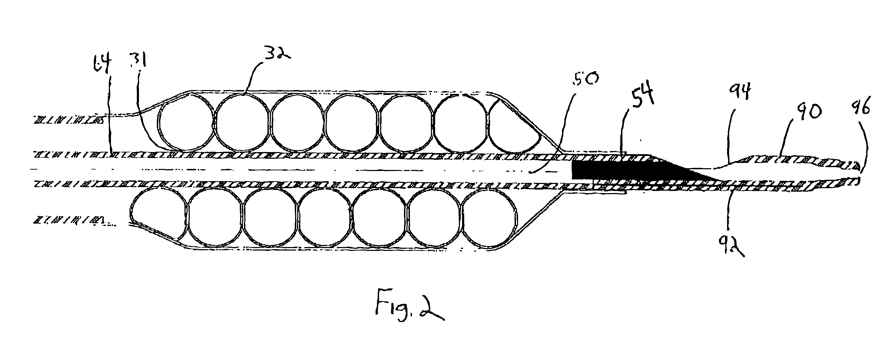

Referring to the drawings wherein like numerals indicate the same elements throughout the views, there is shown in Figure 1 a catheter 2 made in accordance with the present invention. Catheter 2 is designed to be inserted into a vessel of a patient. Catheter 2 has a proximal end 12 (not shown) and a distal end 14. Catheter 2 has an elongated flexible outer shaft 10 and an elongated flexible inner shaft 60. Inner shaft 60 extends through the outer shaft 10 and has a central lumen 50 extending therethrough. Shaft 60 has a proximal portion 62 and a distal portion or distal end 64. Distal portion 64 is designed to be centered within the vessel of the patient. Distal end 14 of catheter 2 includes an inflatable outer balloon 20 and an inflatable inner balloon 30, disposed within outer balloon 20. Distal end 64 of shaft 60 extends through inner balloon 30, and inner balloon 30 is capable of centering distal end 64 within a body vessel once it is inflated. Figures 1 and 2 show the inner balloon 30 as a spiral balloon catheter which surrounds inner shaft 60.

As seen in the figures, and as will be appreciated by those skilled in the art, the inner and outer balloon assembly is a separate piece which is attached to shaft 10. The balloon assembly includes distal portion 64 of shaft 60, which is inserted into proximal portion 62 of shaft 60, to form the entire inner shaft 60. Outer balloon 20 has annular proximal portion 22 which is fitted over shaft 10. Proximal portion 22 is sealed to shaft 10 by any suitable means known in the art, such as heat sealing or adhesive sealing, so that the seal can withstand ordinary angioplasty pressures. Outer balloon 20 is preferably a substantially non-compliant PTCA dilation balloon, and made from any suitable material known in the art, including nylon. Such PTCA balloons are disclosed in the hereinbefore incorporated U.S. Patent 5,304,197.

Inner balloon 30 can be made from any number of substantially non-compliant materials known in the art such as nylon or any number of substantially compliant materials known in the art such as silicon, latex and polyurethane. As mentioned above, inner balloon 30 is preferably a spiral balloon. It can be molded into a spiral shape by blow molding, injection molding etc. Alternatively, the balloon can be manufactured as a non-spiral balloon and then wrapped around central shaft 60 so as to have a spiral or helical configuration. Inner balloon 30 is designed to center distal end 64 of shaft 60 within the vessel of a patient. The spiral balloon has an inner surface 31 which is oriented so as to make contact with distal portion 64, and outer surface 32 which is oriented so as to make contact with outer balloon 20 and, consequently, the wall of the vessel. When balloon 30 is inflated, surface 32 pushes against the wall of the vessel, while surface 31 pushes against distal portion 64. This action causes distal end 64 of shaft 60, and hence central lumen 50 extending therethrough, to be centered within the vessel.

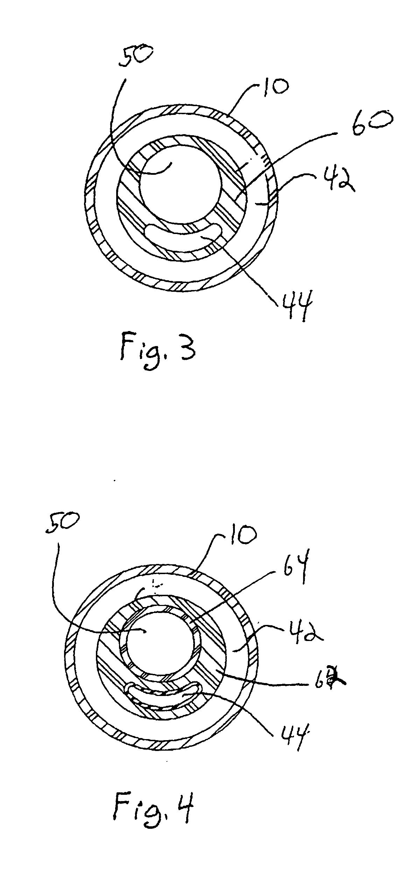

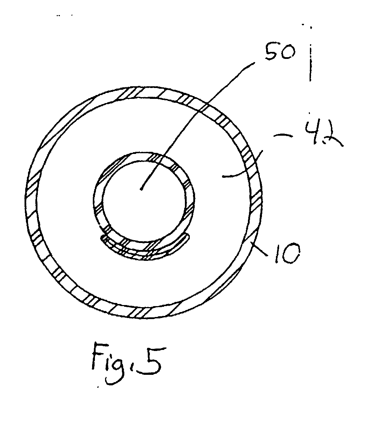

Catheter 2 includes inflation lumens for inflating the two balloons. Preferably, the catheter has first and second inflation lumens extending through at least one of the inner and outer shafts. The outer balloon is in fluid communication with the first inflation lumen for inflation and deflation thereof. The inner balloon is in fluid communication with the second inflation lumen for inflation and deflation thereof. A preferred embodiment of the inflation lumens can be described by referring to Figures 3 and 4. As can be seen first inflation lumen 42 is an annular lumen and is in fluid communication with outer balloon 20. Second inflation lumen 44 extends through central shaft 60, which is a dual lumen shaft. Figures 3 and 4 also show central lumen 50 which, as described below, is for the insertion of a radioactive source wire. Lumens 44 and 50 could be co-axial or concentric. Figures 3 and 4 show that the central lumen is not necessarily centered within shaft 60 at the proximal portion 62. However, at the distal end 64 of shaft 60 the central lumen 50 is centered within shaft 60. The inflation lumen 44 is replaced by the inner balloon 30 itself and therefore is no longer needed within shaft 60. Therefore the arrangement of shaft 60 changes. This can best be described by referring to Figure 5. Figure 5 shows a cross-section of shaft 60 at its distal end, taken along figure 5-5.

Catheter 2 preferably includes a guidewire lumen for receiving a guidewire to help deliver the catheter. The guidewire lumen could simply be a short lumen which is distal of balloons 20 and 30. Such an arrangement is well known in the art and is often referred to as a distal channel guide wire lumen. This feature can best be described by referring to Figure 2. Figure 2 shows the catheter as having a guidewire lumen 90 on the distal end of catheter 2. Guidewire lumen 90 could include a support member 92. Support member 92 is shown as being a small wire located at the bottom and of the lumen. Lumen 90 has an entrance port 94 and an exit port 96 to accommodate a guidewire being placed therethrough. However, if the central lumen 50 is not required to be sealed at its distal end, it could act as the guidewire lumen as well. That is a steerable guidewire would be inserted through the lumen 50 in order to guide the balloon to the diseases site. Thereafter, the steerable guidewire is removed and the radioactive guidewire is inserted.

Central lumen 50 is designed to be centered within the vessel of a patient. The lumen is designed to receive a radioactive source wire. To prevent direct contact between blood and the radioactive source, the radioactive source wire should have a protective sleeve, or lumen 50 should be closed at its distal end, unlike an ordinary guidewire lumen. Figure 2 shows the catheter 2 having a plug 54 which is distal to the spiral balloon 30. Plug 42 can be made from any suitable material such a polymer which is fuses within the lumen 50, or it could comprise an adhesive. An example of a catheter having a closed, radioactive source wire lumen is disclosed in U.S. Patent 5,503,613 issued to Weinberger on April 2, 1996, Central lumen 50 must be large enough to allow for easy maneuvering of a radioactive source wire. Suitable diameters range from 0.010 inch to 0.050 inches. Shaft 60 should be thick enough and strong enough to prevent the radioactive source wire from puncturing through the shaft.

In addition to the present invention providing the advantage of an angioplasty and centering balloon in the same catheter, it also has other advantages. One such advantage is that it allows the manufacture of large centering balloons for use in the peripherals or the like. Currently, it is difficult to manufacture a large spiral non-compliant balloon catheter using blow molding techniques due to the large ration between the peaks and valleys, i.e. major diameter and minor diameter, of the balloon. However, it is practical to manufacture large diameter compliant balloon catheters by wrapping a compliant balloon around a shaft, or tacking/restraining a compliant balloon to a shaft to form a segmented balloon. However, compliant balloons run the risk of over expanding and damaging the vessel wall. With the present invention, the outer balloon can be made non-compliant to help prevent a compliant inner balloon from being over expanded during the centering procedure.

The present invention also allows PTCA procedures and radiation therapy to be performed by the-same catheter. The present invention can be used by inserting the catheter into the vasculature of a patient and directing it to a constricted area through the use of a steerable PTCA guidewire. Thereafter, outer balloon 20 is inflated, thereby compressing the deposits on the artery. Balloon 20 is then deflated or vented and balloon 30 is inflated, so as to center lumen 50 within the vessel. Thereafter, a radioactive source wire can be advanced through lumen 50 so that it is adjacent the diseased area of the vessel. After the vessel receives the requisite amount of radiation, the radioactive source wire is removed. The inner balloon 30 is then deflated and the catheter is removed from the vessel.

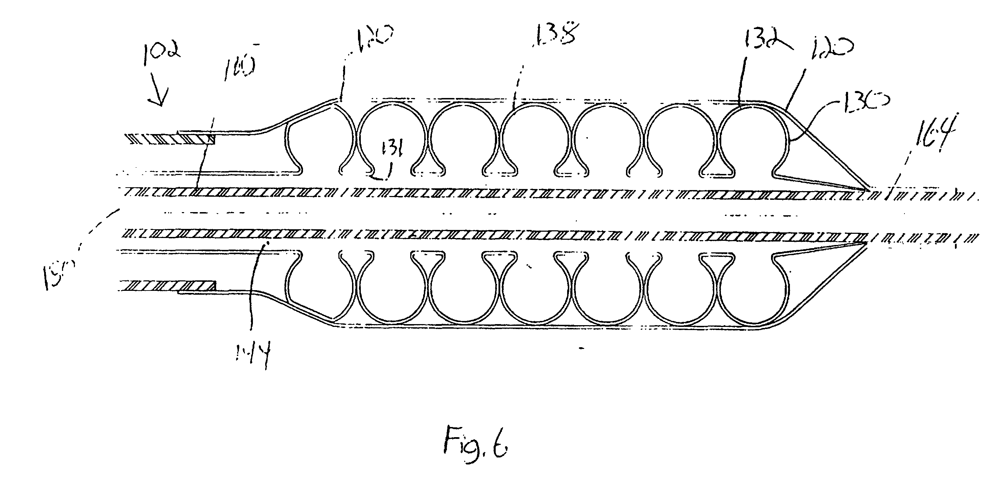

Figures 1 and 2 show the inner balloon 30 as a spiral balloon. However, the inner balloon of the present invention can be a segmented balloon. Figure 6 shows a catheter 102, which is an alternative embodiment of the present invention. Catheter 102 is similar to catheter 2 except that inner centering balloon 130 is a segmented balloon. Balloon 130 is molded, from any suitable materials such as nylon, such that it has a series of semi-circular cross-sectional segments 138 which surround or are concentric with shaft 160. Alternatively, balloon 130 could be made from a compliant material and tacked/restrained to 164. Inflation lumen 144 extends along the length of balloon 130 such that each segment 138 is in fluid communication with the others. Balloon 130 centers the distal end 164 of shaft 160 much in the same way a spiral balloon would. Balloon 130 has an inner surface 131 which makes contact with the fluid in the inflation lumen 144, and outer surface 132 which is oriented so as to make contact with outer balloon 120 and, consequently, the wall of the vessel. When balloon 130 is inflated, surface 132 pushes against the wall of the vessel, while surface 131 pushes against the fluid in lumen 144 which in turn pushes against distal portion 164. This action causes distal end 164 of shaft 160, and hence central lumen 150 extending therethrough, to be centered within the vessel.

Another alternative embodiment of the catheter of the present invention is shown in Figure 7. Figure 7 shows catheter 202, which is an alternative embodiment of the present invention. Catheter 202 is similar to catheter 2 except that inner centering balloon 230 is a multiple axial chamber balloon. Balloon 230 is molded, from any suitable materials such as nylon, latex, etc. such that it has a series of axial segments 238 which extend substantially parallel to shaft. Balloon 230 centers the distal end 264 of shaft 260 much in the same way a spiral balloon would. Segments 238 have an inner surfaces 231 which make contact with distal portion 264, and outer surfaces 232 which are oriented so as to make contact with outer balloon 220 and, consequently, the wall of the vessel. When balloon 230 is inflated, surfaces 232 push against the wall of the vessel, while surfaces 231 push against the distal portion 164. This action causes distal end 264 of shaft 260, and hence central lumen 250 extending therethrough, to be centered within the vessel.

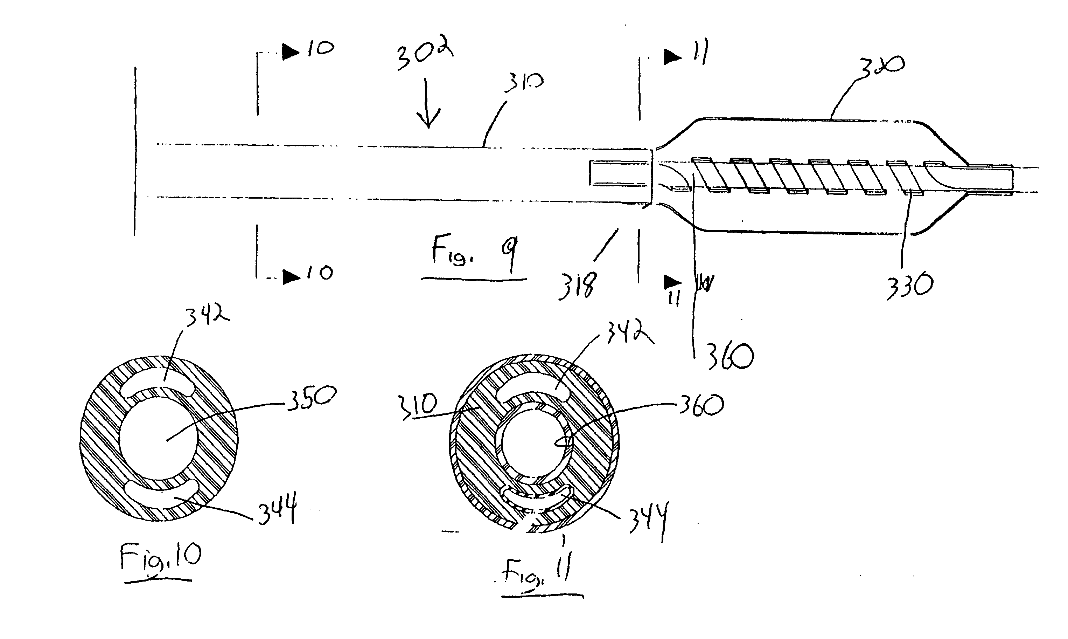

Another alternative embodiment of the catheter of the present invention is shown in Figures 9-11. Figures 9-11 show a catheter 302 which is similar to catheter 2. For the embodiments described above, all of the catheters had an inner which extended through most of the length of the outer shaft. However, the present invention could be made using a single multi-lumen flexible shaft proximal to balloons 320 and 330, and having the inner shaft extend distally therefrom. As seen from Figure 10, flexible outer shaft 310 has a central lumen 350, a first inflation lumen 342, and a second inflation lumen 344, all extending therethrough. As seen from Figure 11, inner shaft 360 extends from the distal end 318 of outer shaft 310, and into balloon 320. In this embodiment, the inner shaft need not extend through the outer shaft.

Although particular embodiments of the present invention have been shown and described, modification may be made to the catheter without departing from the scope of the present invention, which is defined by the following claims.

## Claims

1. A catheter (2) for insertion into a vessel of a patient, said catheter having distal and proximal ends (14, 12), said catheter (2) comprising:
a) an elongated flexible outer shaft (10), an elongated flexible inner shaft (60), said inner and outer shafts (60, 10) having distal and proximal ends, said inner shaft (60) having a central lumen (50) extending therethrough;
b) first and second inflation lumens (42, 44) extending through at least one of said inner and outer shafts (60, 10);
c) an inflatable outer balloon (20) disposed at said distal end (14) of said catheter (2), said outer balloon (20) being in fluid communication with said first inflation lumen (42) for inflation and deflation thereof; and
d) an inflatable inner balloon (30) disposed within said outer balloon (20), said inner balloon (30) being in fluid communication with said second inflation lumen (44) for inflation and deflation thereof, said inner shaft (60) extending through said inner balloon (30);
**characterised in that** said inner balloon (30) is a centering balloon which is able to substantially center said distal end of said inner shaft (60) within said vessel once said inner balloon (30) is inflated.

2. The catheter (2) according to claim 1, wherein said inner shaft (60) is sealed at its distal end.

3. The catheter (2) according to claim 1 or 2, further including a guidewire lumen (90).

4. The catheter (2) according to claim 3 wherein said guidewire lumen (90) comprises a tubular channel disposed distally of said inner and outer balloons (30,20), said channel adapted to receive a guidewire.

5. The catheter (2) according to any one of claims 1 to 4, wherein said inner shaft (60) extends through said outer shaft (10).

6. The catheter (2) according to claim 5, wherein said first inflation lumen (42) is an annular concentric lumen disposed between said inner and outer shafts (60, 10).

7. The catheter (2) according to claim 5 or claim 6, wherein said second inflation lumen (44) is disposed within said inner shaft (60).

8. The catheter (2) according to any one of claims 1 to 7, wherein said inner balloon (30) is a spiral balloon surrounding a predetermined portion of said distal end (64) of said inner shaft (60).

9. The catheter (2) according to any one of claims 1 to 7, wherein said inner balloon (30) comprises a segmented balloon (130) having a plurality of annular segments (138), said plurality of segments (138) surrounding a predetermined portion of said distal end (64) of said inner shaft (60).

10. The catheter (2) according to any one of claims 1 to 7, wherein said inner balloon (30) comprises a plurality of longitudinal segments (238), wherein each segment (238) is substantially parallel to said inner shaft (60), said plurality of segments (238) substantially surrounding a predetermined portion of said distal end (64) of said inner shaft (60).

## Patentansprüche

1. Katheter (2) zur Einführung in ein Gefäß eines Patienten, wobei der Katheter ein distales und ein proximales Ende (14, 12) hat und umfaßt:
a) einen langgestreckten flexiblen äußeren Schaft (10), einen langgestreckten flexiblen inneren Schaft (60), wobei der innere und der äußere Schaft (60, 10) jeweils ein distales und proximales Ende sowie der innere Schaft (60) einen durchgehenden zentralen Hohlraum (50) haben;
b) einen ersten und einen zweiten Aufblas-Hohlraum (42, 44), die sich durch den inneren und den äußeren Schaft (60, 10) erstrecken;
c) einen aufblasbaren äußeren Ballon (20) angeordnet am distalen Ende (14) des Katheters (2), wobei dieser äußere Ballon (20) in Fluid-Verbindung mit dem ersten Aufblas-Hohlraum (42) steht, um diesen Aufzublasen und zu Entleeren und
d) einen aufblasbaren inneren Ballon (30) angeordnet im äußeren Ballon (20), wobei dieser innere Ballon (30) in Fluid-Verbindung mit dem zweiten Aufblas-Hohlraum (44) steht, um diesen Aufzublasen und zu Entleeren und wobei sich der innere Schaft (60) durch den inneren Ballon (30) erstreckt;
**dadurch gekennzeichnet, daß** der innere Ballon (30) ein Zentrier-Ballon ist, welcher das distale Ende des inneren Schaftes (60) in dem Gefäß im wesentlichen zu zentrieren vermag, wenn der innere Ballon (30) aufgeblasen ist.

2. Katheter (2) nach Anspruch 1, bei welchem der innere Schaft (60) an seinem distalen Ende dicht verschlossen ist.

3. Katheter (2) nach Anspruch 1 oder 2, welcher weiterhin einen Führungsdraht-Hohlraum (90) aufweist.

4. Katheter (2) nach Anspruch 3, bei welchem der Führungsdraht-Hohlraum (90) aus einem rohrartigen Kanal besteht, der distal vom inneren und vom äußeren Ballon (30, 20) angeordnet ist und der einen Führungsdraht aufzunehmen vermag.

5. Katheter (2) nach einem der Ansprüche 1 bis 4, bei welchem sich der innere Schaft (60) durch den äußeren Schaft (10) erstreckt.

6. Katheter (2) nach Anspruch 5, bei welchem der erste Einblas-Hohlraum (42) ein ringförmiger konzentrischer Hohlraum zwischen dem inneren und dem äußeren Schaft (60, 10) ist.

7. Katheter (2) nach Anspruch 5 oder 6, bei welchem der zweite Aufblas-Hohlraum (44) im inneren Schaft (60) angeordnet ist.

8. Katheter (2) nach einem der Ansprüche 1 bis 7, bei welchem der innere Ballon (30) ein Spiral-Ballon ist, welcher einen vorgegebenen Teil des distalen Endes (64) des inneren Schaftes (60) umgibt.

9. Katheter (2) nach einem der Ansprüche 1 bis 7, bei welchem der innere Ballon (30) ein segmentierter Ballon (130) mit einer Vielzahl ringförmiger Segmente (138) ist, welche einen vorgegebenen Teil des distalen Endes (64) des inneren Schaftes (60) umgeben.

10. Katheter (2) nach einem der Ansprüche 1 bis 7, bei welchem der innere Ballon (30) eine Vielzahl von Längs-Segmenten (238) umfaßt, wobei jedes Segment (238) im wesentlichen parallel zum inneren Schaft (60) verläuft, und diese Vielzahl der Segmente (238) einen vorgegebenen Teil des distalen Endes (64) des inneren Schaftes (60) umgibt.

## Revendications

1. Cathéter (2) destiné à être introduit dans un vaisseau d'un patient, ledit cathéter présentant une extrémité proximale et une extrémité distale (14,12), ledit cathéter comportant :
a) un conduit extérieur allongé et flexible (10), un conduit intérieur allongé et flexible (60), lesdits conduits extérieur et intérieur (60, 10) présentant respectivement une extrémité distale et une extrémité proximale, ledit conduit intérieur (60) présentant une lumière (50) qui s'étend sur toute sa longueur ;
b) une première lumière de gonflage (42) et une seconde lumière de gonflage (44) s'étendant sur toute la longueur d'au moins un desdits conduits intérieur et extérieur (60, 10) ;
c) un ballon gonflable externe (20) disposé à ladite extrémité distale (14) dudit cathéter (2), ledit ballon (20) étant en communication fluidique avec ladite première lumière de gonflage (42) pour effectuer le gonflage et le dégonflage dudit ballon gonflable externe (20) ; et
d) un ballon gonflable interne (30) disposé à l'intérieur dudit ballon externe (20), ledit ballon interne (30) étant en communication fluidique avec ladite seconde lumière de gonflage (44) pour le gonflage et le dégonflage dudit ballon gonflable interne (30), ledit conduit intérieur (60) traversant d'un bout à l'autre l'espace occupé par ledit ballon interne (30) ;
**caractérisé en ce que** ledit ballon interne (30) est un ballon de centrage propre à assurer le centrage effectif de la position de ladite extrémité distale dudit conduit intérieur (60) dans ledit vaisseau une fois que ledit ballon interne (30) est gonflé.

2. Cathéter (2) selon la revendication 1, dans lequel ledit conduit intérieur (60) est occlus à son extrémité distale.

3. Cathéter (2) selon l'une quelconque des revendications 1 et 2, comprenant de plus une lumière (90) pour fil de guidage.

4. Cathéter (2) selon la revendication 3 dans lequel ladite lumière (90) pour fil de guidage présente un canal tubulaire disposé à l'extrémité distale desdits ballons interne et externe (30, 20), ledit canal étant à même de recevoir un fil de guidage.

5. Cathéter (2) selon l'une quelconque des revendications 1 à 4, dans lequel ledit conduit intérieur (60) traverse d'un bout à l'autre ledit conduit extérieur (10).

6. Cathéter (2) selon la revendication 5, dans lequel ladite première lumière de gonflage (42) est de forme annulaire, concentriquement disposée entre lesdits conduits intérieur et extérieur (60, 10).

7. Cathéter (2) selon la revendication 5 ou la revendication 6, dans lequel ladite seconde lumière de gonflage (44) est située à l'intérieur dudit conduit intérieur (60).

8. Cathéter (2) selon l'une quelconque des revendications 1 à 7, dans lequel ledit ballon interne (30) forme une spirale enroulée sur une portion prédéfinie de ladite extrémité distale (64) dudit conduit intérieur (60).

9. Cathéter (2) selon l'une quelconque des revendications 1 à 7, dans lequel ledit ballon interne (30) est un ballon segmenté (130) constitué d'une pluralité de segments annulaires (138) entourant une portion définie de ladite extrémité distale (64) dudit conduit intérieur (60).

10. Cathéter (2) selon l'une quelconque des revendications 1 à 7, dans lequel ledit ballon interne (30) comporte une pluralité de segments longitudinaux (238), dans lequel chacun des segments (238) est disposée parallèlement audit conduit intérieur (60), ladite pluralité de segments (238) entourant de façon effective une portion prédéfinie de ladite extrémité distale (64) dudit conduit intérieur (60).
